# EUROPÄISCHE PATENTSCHRIFT

(11) **EP 3 659 558 B1**
(45) Veröffentlichungstag und Bekanntmachung des Hinweises auf die Patenterteilung: **19.01.2022**
(21) Anmeldenummer: 18209621.4
(22) Anmeldetag: 30.11.2018
(51) Int. Cl.: A61F 2/50, A61F 2/54, A61F 2/60, A61F 2/66, A61F 2/76

(54) **SENSORTRÄGER ZUM ANORDNEN AN EINER PROTHESE**
SENSOR HOLDER FOR FITTING TO A PROSTHESIS
SUPPORT DE CAPTEUR DESTINÉ À ÊTRE AGENCÉ SUR UNE PROTHÈSE

(43) Veröffentlichungstag der Anmeldung: 03.06.2020
(73) Patentinhaber: Saphenus Medical Technology GmbH, 3500 Krems an der Donau (AT)
(72) Erfinder: ZACH, Bernhard, 1220 Wien (AT); BRANDSTÄTTER, Martin, 1090 Wien (AT); PITSCHL, Aaron, 1220 Wien (AT); SCHULTHEIS, Rainer, 1040 Wien (AT); MÜHLENBEREND, Andreas, 99423 Weimer (DE)
(74) Vertreter: Patentbüro Paul Rosenich AG

(56) Entgegenhaltungen:
- WO-A1-2018/089543
- DE-A1-102009 030 995
- DE-A1-102015 006 515
- US-A1- 2016 206 242

## Beschreibung

Die Erfindung betrifft einen Sensorträger zum Anordnen an einer Prothese, nach dem Oberbegriff des Anspruchs 1.

Moderne Prothesen verfügen oftmals über Sensoren. Typischerweise werden Sensoren direkt innerhalb einer Prothese angeordnet und sind darin fest verbaut. Beispielsweise werden innerhalb der Prothesensohle einer Fussprothese mehrere Drucksensoren verbaut. Die Prothese weist zusätzlich oftmals mehrere Stimulatoren auf. Diese Stimulatoren sind mit mehreren Drucksensoren elektrisch verbunden. Die Stimulatoren werden somit von den Drucksensoren gesteuert bzw. angeregt und wirken über die Haut auf die verbleibende Extremität des Patienten. Dadurch ermöglichen die Stimulatoren dem Prothesenbenutzer einen Druck auf den Drucksensoren wahrzunehmen und den Druck an der Prothesensohle somit "zu fühlen". Derartige Vorrichtungen werden beispielsweise in der WO 98/25552 A1 offenbart.

Nachteilig an dieser bekannten Lösung ist, dass derartige Prothesensysteme einerseits sehr teuer sind und andererseits die Anpassungsphase und Eingewöhnungsphase für einen Prothesenbenutzer an die Prothese sehr zeitintensiv sind. Ausserdem sind diese mit mehreren Terminen bei einem Facharzt und/oder bei einem Orthopäden verbunden. Dort müssen die Positionen der Stimulatoren und der Drucksensoren mehrmals nachjustiert werden, was schwierig sein kann, wenn sie schon fix verbaut sind.

Die WO 2018/089543 A1 offenbart eine mechanische Fussprothese, wobei in einer Ausführungsform ein standardisierter Fussprothesenträger - eine Socke, ein Schuh - vorgesehen ist, an dem mehrere Drucksensoren entlang der Supportfläche des Fussprothesenträger angeordnet sind. Die mehreren Drucksensoren sind mit einer Prothesensteuerungseinrichtung zum Austausch von Sensorsignalen verbunden.

Es ist die Aufgabe der vorliegenden Erfindung, einen oder mehrere Nachteile des Standes der Technik zu beheben. Insbesondere soll ein Sensorträger geschaffen werden, welcher an bestehenden Prothesen einfach angebracht werden kann, ohne diese baulich zu verändern. Der Sensorträger soll an der Prothese im angeordneten Zustand zuverlässig funktionieren und andererseits auch wieder entfernt und z.B. an einer anderen Prothese wieder einfach montierbar sein.

Die Aufgabe wird durch die kennzeichnenden Merkmale des unabhängigen Anspruchs gelöst. Vorteilhafte Weiterbildungen sind in der Beschreibung, den Figuren, der Figurenbeschreibung und in den abhängigen Patentansprüchen dargelegt.

Ein erfindungsgemässer Sensorträger zum Anordnen an einer Prothese, umfasst einen Grundkörper zum Aufnehmen von Sensoren, wobei der Grundkörper einen Sensorabschnitt mit Sensoren aufweist, und der Grundkörper einen Halteabschnitt zum Halten des Grundkörpers an einem Prothesenteil aufweist. Der Grundkörper weist einen gestrickten Gewebeschlauch auf. Der Halteabschnitt weist eine erste Strickstruktur auf, welche ein sitzfestes Halten des Grundkörpers am Prothesenteil ermöglicht. Der Sensorabschnitt weist eine davon unterschiedliche Strickstruktur auf, welche ein sicheres Positionieren der Sensoren relativ zum Prothesenteil ermöglicht.

Der vorliegende Sensorträger kann an jeder beliebigen Prothese angeordnet werden, welche somit selbst keine Sensoren aufweisen muss und daher einfacher konstruiert sein kann. Dabei wird mithilfe der unterschiedlichen Strickstruktur am Sensorabschnitt und am Halteabschnitt des Grundkörpers einerseits die Sensoren im Grundkörper fixiert und andererseits die Sensoren an einer zuvor definierten Position am Prothesenteil fixiert. Die Strickstruktur am Haftabschnitt verhindert das Bilden von Falten im Gewebeschlauch. Eine Faltenbildung könnte ein Fehlpositionieren der Sensoren an der Prothese bewirken. Die Strickstruktur am Sensorabschnitt ist vergleichsweise widerstandfähiger gestrickt und minimiert somit die mechanische Belastung der Sensoren beim Benutzen der Prothese, wodurch deren Lebensdauer erhöht wird und auch die Prothese selbst weniger Stossbelastet wird (Dämpfung).

Der neuartige Sensorträger ermöglicht dem Prothesenbenutzer die Prothesenbewegung/Abrollbewegung des Prothesenfusses genauso "zu fühlen", wie mit herkömmlichen Prothesen mit eingebauten Sensoren, jedoch erlaubt die einfache Entfernbarkeit der Sensoren zusammen mit dem Sensorträger eine einfache Reinigung der Prothese sodass der tägliche Umgang mit der Prothese verbessert wird.

Auch schwierige Bewegungen, beispielsweise das Bewegen/Gehen im steilen Gelände mit einem Kunstfuss, kann mittels dem neuartigem Sensorträger erlernt werden. Da der vorliegende Sensorträger an jede beliebige Prothese angeordnet werden kann, erspart sich der Prothesenbenutzer einen Prothesentausch, wenn der Prothesenbenutzer eine "fühlende" Prothese verwenden möchte. Da ein Prothesentausch für einen Prothesenbenutzer sehr unangenehm ist, weil der Prothesenschaft an die Anatomie der verbleibenden Extremität des Prothesenbenutzers angepasst werden muss, ist die Erfindung von grossem Vorteil.

Insbesondere ist der Sensorträger geeignet, um an einer Kunsthand oder einem Kunstfuss angeordnet zu werden. Dabei handelt es sich um weitverbreitete Prothesenarten, welche somit mit dem hier vorliegend beschriebenen Sensorträger verbessert werden können.

Vorzugsweise weist der Halteabschnitt neben seiner ersten Strickstruktur eine zweite Strickstruktur auf. Die Strickstrukturen am Halteabschnitt ermöglichen ein sitzfestes Halten an dem Prothesenteil. Diese sind an unterschiedlichen Positionen am Grundkörper angeordnet, wodurch dieser an mehr als einer Position stabil mit dem Prothesenteil haltend zusammenwirkt.

Bevorzugt ist die zweite Strickstruktur unterschiedlich zur ersten Strickstruktur ausgebildet. Unterschiedliche Strickstrukturen weisen unterschiedliche Dehnungseigenschaften auf, wodurch der Sensorträger zuverlässig am Prothesenteil angeordnet werden kann.

Insbesondere ist die zweite Strickstruktur formstabil zum Umfassen eines Prothesenteils ausgebildet. Eine formstabile Strickstruktur bewirkt einen verbesserten Halt am Prothesenteil, da die Strickstruktur kraftschlüssig mit dem Prothesenteil verbunden ist. Beispielsweise ist die formstabile Strickstruktur an jenem Bereich des Sensorträgers angeordnet, der mit der Ferse eines Kunstfusses zusammenwirkt. Dies ermöglicht dem Prothesenbenutzter das verbesserte "Fühlen" einer Bewegungsabfolge, beispielsweise beim Gehen oder Laufen, wodurch das Vertrauen des Prothesenbenutzers in die Prothese und in der Folge die Gehsicherheit erhöht wird.

Bevorzugterweise weist der Halteabschnitt neben seiner ersten Strickstruktur eine dritte Strickstruktur auf, welche unterschiedlich zur ersten Strickstruktur und zur zweiten Strickstruktur ausgebildet ist. Damit werden die Halteeigenschaften des Sensorträgers an der Prothese weiter verbessert, indem eine Faltenbildung im gestrickten Gewebeschlauch verhindert wird.

Insbesondere ist die dritte Strickstruktur elastisch ausgebildet. Der gestrickte Gewebeschlauch kann eine Vorspannung aufweisen und kann mithilfe der elastischen dritten Strickstruktur einfach auf den Prothesenteil aufgespannt werden.

Vorzugsweise weist die erste Strickstruktur des Halteabschnitts eine erste Fadenstärke auf und die zweite Strickstruktur des Halteabschnitts eine zweite Fadenstärke auf. Die hier beschriebenen unterschiedlichen Strickstrukturen weisen unterschiedliche Funktionalitäten auf, welche einerseits über die Strickstruktur selbst, aber auch mithilfe der unterschiedlichen Fadenstärken einstellbar sind. Die unterschiedlichen Funktionalitäten der Strickstruktur sind an unterschiedlichen Abschnitten an der Prothese vorteilhaft. Mit den unterschiedlichen Fadenstärken können die Halteeigenschaften von gleichen Strickstrukturen verbessert werden. Beispielsweise ist eine rautenförmige Strickstruktur vorteilhaft, da diese vorteilhafte Dehnungseigenschaften aufweisen. Insbesondere liegt die Fadenstärke der ersten Strickstruktur in einem Bereich zwischen 0.1 mm und 0.6 mm, vorteilhaft bei 0.3 mm. Insbesondere liegt die Fadenstärke der zweiten Strickstruktur in einem Bereich zwischen 0.5 mm und 1.5 mm, vorteilhaft bei 1 mm.

Bevorzugt weist die dritte Strickstruktur des Halteabschnittes eine dritte Fadenstärke auf. Eine dritte Strickstruktur mit einer dritten Fadenstärke erhöht die Flexibilität des Einsatzbereiches des Sensorträgers.

Insbesondere liegt die Fadenstärke der dritten Strickstruktur in einem Bereich zwischen 0.3 mm und 0.9 mm, vorteilhaft bei 0.6 mm.

Bevorzugterweise weist die Strickstruktur des Sensorabschnitts eine Fadenstärke auf, welche unterschiedlich zur ersten Fadenstärke des Halteabschnitts ist. Damit werden die für den Sensorabschnitt spezifischen Eigenschaften der Strickstruktur verbessert. Beispielsweise ist die Fadenstärke der Strickstruktur des Sensorabschnitts dicker als jene am Halteabschnitt, wodurch die Sensoren im Sensorabschnitt vor unerwünschten mechanischen Belastungen geschützt werden. Insbesondere liegt die Fadenstärke der Strickstruktur des Sensorabschnitts in einem Bereich zwischen 0.6 mm und 1.5 mm, vorteilhaft bei 0.8 mm.

Vorzugsweise ist zumindest eine der Strickstrukturen aus einer widerstandsfähigen Kunstfaser hergestellt. Kunstfasern sind langlebige und robuste Materialien, welche mithilfe eines Strickverfahrens verarbeitbar sind. Beispielsweise wird eine Kunstfaser aus Nylon oder Polyester oder Materialien mit vergleichbaren Eigenschaften verwendet. Diese verhindern, dass sich in gezielten Bereichen der gestrickte Gewebeschlauch ausdehnt oder ausleiert kann. Besonders im Bereich einer Prothesensohle eines Kunstfusses, soll eine Dehnung und Stauchung verhindert werden, um die Sensoren vor Beschädigung durch Zugkräfte zu schützen.

Bevorzugterweise ist der Sensorabschnitt mehrlagig ausgebildet und weist eine Sensorschicht auf, in der die Sensoren eingebettet sind. Die Sensorschicht ermöglicht ein strukturiertes Anordnen der Sensoren im Sensorträger, wobei dabei individuelle Bedürfnisse des Prothesenbenutzers berücksichtig werden können. Der mehrlagige Aufbau des Sensorabschnitts führt zu einem verbesserten Schutz der Sensoren und addiert gleichzeitig zur Dämpfungswirkung zwischen der Prothese und der Umgebung (z.B. des Untergrunds oder eines Innenschuhs).

Bevorzugt umfassen die Sensoren - wie an sich bekannt - zumindest einen Drucksensor. Der zumindest eine Drucksensor ist dafür ausgebildet, Drücke, welche normalerweise direkt auf die Prothese wirken, aufzunehmen und in ein elektrisches Signal umzuwandeln. Das elektrische Signal wird anschliessend weitergeleitet. Beispielsweise ist der zumindest eine Drucksensor piezoelektrisch aufgebaut.

Alternativ oder ergänzend umfassen die Sensoren zumindest einen Kraftsensor. Der Kraftsensor ist dafür ausgebildet, Kräfte, welche normalerweise direkt auf die Prothese wirken, aufzunehmen und in ein elektrisches Signal umzuwandeln, welches anschliessend weitergeleitet wird. Beispielsweise wird als Kraftsensor ein Dehnmessstreifen verwendet, da dieser leicht ist und platzsparend in die Sensorschicht angeordnet werden kann. Darüber hinaus sind Kraftsensoren ausgebildet, neben Druckkräfte auch Scherkräfte zu messen.

Vorzugsweise ist an dem Sensorabschnitt zusätzlich zur Strickstruktur eine im Anwendungszustand an einem Prothesenteil abgewandte, äussere Schutzschicht vorgesehen. Die äussere Schutzschicht schützt die Sensorschicht vor unerwünschten mechanischen Belastungen, welche beispielsweise zur Faltenbildung der Sensorschicht führen können. Der Sensorträger kann so nicht nur direkt an der Prothese angeordnet werden, sondern auch mit der Prothese innerhalb eines Bekleidungsstücks, wie einen Schuh oder einen Handschuh, eingesetzt werden, was ohne die verbesserte Massnahme zu einer Faltenbildung an der Sensorschicht führen könnte. Zusätzlich erhöht diese äussere Schutzschicht die Lebensdauer der Sensorschicht, wenn der Benutzer beispielsweise ohne zusätzliche Schutzmassnahmen am Untergrund bzw. am Boden geht.

Bevorzugt ist eine dem Prothesenteil zugewandte innere Schutzschicht vorgesehen. Die innere Schutzschicht ist dazu ausgebildet, die Sensorschicht nach Innen hin mechanisch zu schützen, wenn beispielsweise der Sensorträger auf die Prothese aufgespannt wird. Durch das Aufspannen des Sensorträgers auf die Prothese können nämlich Dehnungskräfte von der inneren Schutzschicht aufgenommen werden, welche somit von der Sensorschicht ferngehalten werden können.

Bevorzugterweise ist die äussere Schutzschicht zumindest in eine Richtung rutschhemmend ausgebildet. Damit wird das Verrutschen der äusseren Schutzschicht gegenüber einem Untergrund oder einem Innenschuh verhindert und somit auch das Verrutschen der Sensorschicht im Sensorträger unterbunden. Als äussere Schutzschicht kann beispielsweise Kunstleder, wie Alcantara, verwendet werden. Beispielsweise ist die Oberfläche der äusseren Schutzschicht rutschhemmend.

Insbesondere ist die äussere Schutzschicht zumindest abschnittsweise oder als Ganzes aus einem rutschhemmenden Material gefertigt. Ein rutschhemmendes Material verhindert ein Verrutschen des Sensorträgers beim Bewegen der Prothese, welche sich samt Sensorträger in einem Bekleidungsstück, wie beispielsweise einen Schuh, befindet. Dabei wirkt das rutschhemmende Material mit dem Material des Bekleidungsstücks haftreibend zusammen.

Das rutschhemmende Material kann aber auch als Beschichtung auf einem Trägermaterial aufgebracht werden. Dabei kann das Trägermaterial formstabil sein und nur die die Beschichtung rutschhemmend ausgebildet sein. Beispielsweise kann als Trägermaterial ein Kunstleder verwendet werden, welches eine Beschichtung aus Silikon, oder vergleichbar Materialien, aufweist. Silikon lässt sich einfach und grossflächig auf ein Trägermaterial aufbringen und weist eine hohe Haftstabilität auf, sodass ein zeitnaher Abrieb der Beschichtung verhindert werden kann.

Alternativ oder ergänzend ist die äussere Schutzschicht zumindest abschnittsweise aus einem rutschfördernden Material gefertigt. Ein rutschförderndes Material erlaubt das einfache Rutschen der äusseren Schutzschicht zumindest in eine Richtung, sodass beispielsweise das Anordnen des Sensorträgers innerhalb eines Kleidungsstücks einfach ausführbar ist. Dabei wirkt das rutschfördernde Material mit dem Bekleidungsstück gleitreibend (wenigstens in eine Richtung) zusammen.

Insbesondere ist die äussere Schutzschicht zumindest in eine Richtung rutschfördernd ausgebildet. Damit wird das Anordnen des Sensorträgers innerhalb eines Bekleidungsstücks vereinfacht und die Bildung von Falten am Sensorträger verhindert. Das rutschfördernde Material kann auch als Beschichtung auf ein Trägermaterial aufgebraucht werden. Dabei kann das Trägermaterial formstabil sein und die Beschichtung rutschfördernd sein. Beispielsweise kann als Trägermaterial ein Kunstleder verwendet werden, welches eine Beschichtung aus Vinyl, wie eine Vinylfolie, oder aus vergleichbaren Materialien, aufweist. Eine Vinylfolie lässt sich einfach und grossflächig auf ein Trägermaterial aufbringen und weist eine hohe Haftstabilität auf, sodass ein zeitnaher Abrieb der Beschichtung verhindert werden kann.

Bevorzugt weist die äussere Schutzschicht eine Profilstruktur zum Verbessern der rutschhemmenden oder rutschfördernden Eigenschaft auf. Als eine Profilstruktur, wie hier vorliegend beschrieben, wird eine strukturierte Anordnung von Materialien unterschiedlicher Eigenschaften verstanden, welche in einem strukturierten Abstand zueinander angeordnet werden und miteinander zusammenwirken, um die rutschhemmenden Eigenschaften in eine Richtung oder rutschfördernden Eigenschaft in eine andere Richtung des Sensorträgers zu verbessern. Beispielsweise kann es sich dabei um eine glatte fellartige Struktur handeln.

Alternativ oder ergänzend ist die innere Schutzschicht zumindest in eine Richtung rutschhemmend ausgebildet. Damit wird das Verrutschen der inneren Schutzschicht verhindert und somit auch das Verrutschen der Sensorschicht im Sensorträger unterbunden. Das rutschhemmende Material kann als Beschichtung auf ein Trägermaterial aufgebraucht werden. Dabei kann das Trägermaterial formstabil sein und die Beschichtung rutschhemmend sein. Beispielsweise ist, wie zuvor beschrieben, ein Trägermaterial aus Kunstleder verwendbar, welches mit Silikon beschichtet ist.

Bevorzugt ist die innere Schutzschicht zumindest in eine Richtung rutschhemmend ausgebildet. Damit wird das Verrutschen der inneren

Schutzschicht an der Prothese verhindert und somit auch das Verrutschen der Sensorschicht im Sensorträger unterbunden.

Insbesondere ist die innere Schutzschicht zumindest abschnittsweise aus einem rutschhemmenden Material gefertigt. Ein rutschhemmendes Material verhindert ein Verrutschen des Sensorträgers beim Benutzen der Prothese. Dabei wirkt das rutschhemmende Material mit dem Material der Prothese haftreibend zusammen.

Alternativ oder ergänzend ist die innere Schutzschicht zumindest abschnittsweise aus einem rutschfördernden Material gefertigt. Ein rutschförderndes Material erlaubt das einfachere Aufziehen des Sensorträgers auf die Prothese. Das rutschfördernde Material wirkt bei einem solchen Aufbau zumindest in eine Richtung gleitreibend mit der Prothese zusammen.

Insbesondere ist die innere Schutzschicht zumindest in eine Richtung rutschfördernd ausgebildet. Damit wird das Aufziehen des Sensorträgers an der Prothese vereinfacht und die Bildung von Falten am Sensorträger verhindert. Das rutschfördernde Material kann als Beschichtung auf ein Trägermaterial aufgebraucht werden. Dabei kann das Trägermaterial formstabil sein und die Beschichtung rutschfördernd ausgebildet sein. Beispielsweise ist, wie zuvor beschrieben, als Trägermaterial ein Kunstleder verwendbar, welches mit Vinyl beschichtet ist.

Bevorzugt weist die innere Schutzschicht eine Profilstruktur zum Verbessern der rutschhemmenden oder rutschfördernden Eigenschaft auf. Dabei wirkt die Profilstruktur der inneren Schutzschicht in eine Richtung gleitreibend mit der Prothese zusammen und in einer anderen Richtung haftreibend mit der Prothese zusammen.

Alternativ oder ergänzend ist die innere Schutzschicht zumindest in eine Richtung rutschhemmend ausgebildet. Damit wird das Verrutschen der inneren Schutzschicht verhindert und somit auch das Verrutschen der Sensorschicht im Sensorträger unterbunden.

Insbesondere ist die, wie hier vorliegend beschriebene, Profilstruktur geprägt, wodurch die Eigenschaften der Rutschfestigkeit im Sensorabschnitt zusätzlich verstärkt wird, ohne dass damit das Anordnen der Sensorträgers an einem Bekleidungsstück erschwert wird.

Vorzugsweise ist die Sensorschicht zwischen der äusseren Schutzschicht und der inneren Schutzschicht eingebettet, sodass eine allfällige Faltenbildung der Sensorschicht aufgrund einer mechanischen Belastung, verhindert wird.

Insbesondere ist die Sensorschicht separierbar zwischen der äusseren Schutzschicht und der inneren Schutzschicht eingebettet, sodass diese austauschbar ist. Dies ermöglicht das Reinigen des Sensorträgers, ohne die Sensoren selbst in den Reinigungsvorgang miteinzubeziehen.

Bevorzugterweise ist die Sensorschicht stoffschlüssig im Sensorabschnitt angeordnet. Dabei ist die Sensorschicht beispielsweise mit einer ersten Seite an den Sensorabschnitt geklebt, sodass mögliche Scherkräfte über die äussere Schutzschicht oder der inneren Schutzschicht neutralisiert werden können und nicht auf die Sensorschicht übertragen werden.

Vorzugsweise ist eine Steuerungsvorrichtung vorgesehen, welche mit den Sensoren elektrisch verbunden ist. Die Steuereinrichtung empfängt die von den Sensoren erzeugten elektrischen Signale und kann diese weiterverarbeiten. Dabei sind die Sensoren mithilfe von elektrischen Leitungen mit der Steuerungsvorrichtung verbunden. Die elektrischen Leitungen können einerseits separate Leitungen sein oder in einer der Strickstrukturen eingearbeitete, elektrisch leitfähige Fäden sein.

Bevorzugterweise ist die Steuerungsvorrichtung separierbar mit den Sensoren der Sensorschicht verbunden. Dafür kann die Steuerungsvorrichtung einen Anschlussabschnitt aufweisen, an welchen die elektrischen Leitungen lösbar bzw. separierbar angeordnet sind. Die Steuerungsvorrichtung weist gegebenenfalls unterschiedliche Steuerungsprogramme auf, welche beispielsweise hinsichtlich der strukturellen Anordnung der Sensoren in der Sensorschicht abstimmbar ist. Darüber hinaus kann eine Sensorschicht mit unterschiedlichen Steuerungsvorrichtungen betrieben werden. Die Benutzung einer erfindungsgemässen Sensorschicht in oder an einem erfindungsgemässen Sensorträger kann für unterschiedliche Steuerungsvorrichtungen denkbar sein, so dass die Sensorschicht bzw. Der Sensorträger als Modul mit verschiedenen modulartigen Steuerungsvorrichtungen kombinierbar ist.

Vorzugsweise ist die Steuerungsvorrichtung am Prothesenteil befestigbar. Damit kann die Steuerungsvorrichtung separiert vom Sensorträger an dem Prothesenteil oder der Prothese angeordnet werden. Beispielsweise ist der Prothesenteil der Prothesenschaft, womit eine stabile Befestigung der Steuerungsvorrichtung bereitgestellt werden kann. Diese Ausbildung und die Nachfolgenden sind auch unabhängig von den übrigen Markmalen des Sensorträgers erfinderisch und anwendbar.

Bevorzugt ist die Steuerungsvorrichtung am Prothesenteil lösbar befestigbar. Damit wird die Steuerungsvorrichtung stabil gehalten und das Austauschen der Steuerungsvorrichtung für den Prothesenbenutzer vereinfacht.

Vorzugsweise ist die Steuerungsvorrichtung mittels einer Magnethalterung am Prothesenteil befestigbar. Damit wird das lösbare Befestigen der Steuerungsvorrichtung weiter verbessert.

Weitere Vorteile, Merkmale und Einzelheiten der Erfindung ergeben sich aus der nachfolgenden Beschreibung, in der unter Bezugnahme auf die Zeichnungen und die Ausführungsbeispiele der Erfindung beschrieben sind. Aufzählungen wie erste, zweite, dritte oder weitere dienen lediglich zur Identifikation der Bauteile.

Die Bezugszeichenliste ist wie auch der technische Inhalt der Patentansprüche und Figuren Bestandteil der Offenbarung. Die Figuren werden zusammenhängend und übergreifend beschrieben. Gleiche Bezugszeichen bedeuten gleiche Bauteile, Bezugszeichen mit unterschiedlichen Indizes geben funktionsgleiche oder ähnliche Bauteile an.

Die US 2016/206242 A1 offenbart eine Sensorvorrichtung in einem flexiblen und dehnbaren Bekleidungsstück, wobei die darin angeordneten Sensoren physiologische Parameter der unter dem Bekleidungsstück liegenden Haut oder des Gewebes erfassen. Die im Bekleidungsstück angeordneten Sensoren können auch Parameter wie die Kraft oder den Druck erfassen, die auf oder gegen ein Gewebe oder eine darunterliegende Haut ausgeübt werden. Die Sensorvorrichtung weist Drucksensoren und Leitungen auf, die aus leitfähigen Gewebematerial bestehen und direkt in das Bekleidungsstück eingearbeitet sind.

Der Gegenstand der US 2016/206242 A1 mag wie ein vergleichbarer Sensorträger aussehen, weshalb das zuvor genannte Dokument hier erwähnt ist. Allerdings gibt es zwischen diesem bekannten Dokument und der Aufgabenstellung, ebenso wie der Lösung der Aufgabenstellung, keinerlei Motivation den Fachmann sich der Lehre dieses Dokuments zu bedienen. Dies deshalb, weil der Gegenstand dieses Dokuments nichts mit Prothetik zu tun hat.

### Es zeigen dabei:

- Fig. 1: einen erfindungsgemässen Sensorträger in einer perspektivischen Ansicht,
- Fig. 2: eine Sensorschicht des Sensorträgers gemäss Fig. 1 in einer Aufsicht,
- Fig. 3: die Sensorschicht gemäss Fig. 2 in dem Sensorabschnitt des Sensorträgers gemäss Fig. 1 entlang der Schnittansicht III,
- Fig. 4: eine innere Schutzschicht des Sensorabschnitts im Sensorträger gemäss Fig.1 in einer Aufsicht,
- Fig. 5: eine äussere Schutzschicht des Sensorabschnitts im Sensorträger gemäss Fig.1 in einer Aufsicht,
- Fig. 6: den Sensorträger gemäss Fig.1 mit einer Steuerungsvorrichtung in einer Seitenansicht, und
- Fig. 7: den Sensorträger gemäss Fig.1 mit einer Steuerungsvorrichtung an einer Prothese in einer perspektivischen Ansicht.

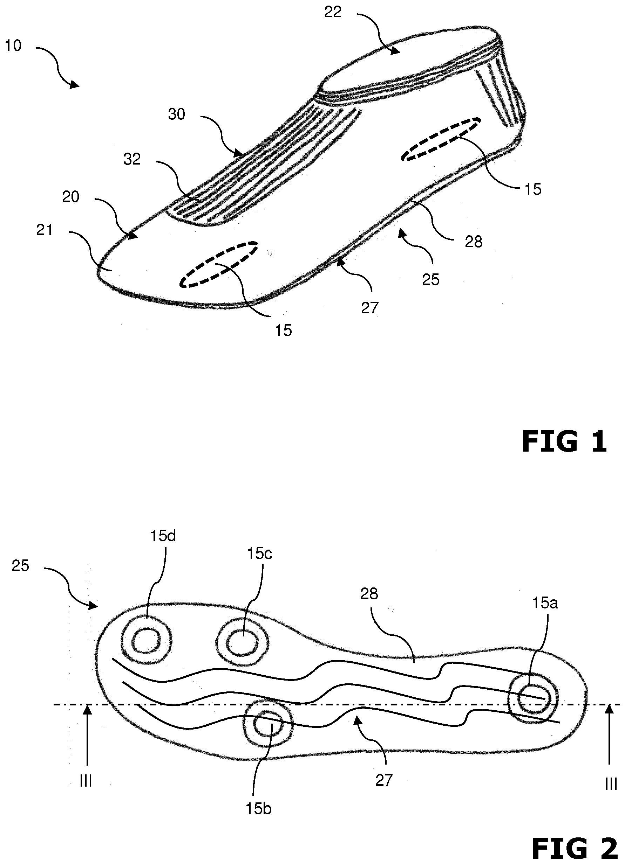

Fig. 1 zeigt den Sensorträger 10 zum Anordnen an einer Prothese bzw. an einem Prothesenteil, wobei die Prothese beispielsweise ein Kunstfuss ist. Der Sensorträger 10 umfasst einen Grundkörper 20, welcher aus einem gestrickten Gewebeschlauch 21 besteht, der an einem (vorderen) Schlauchende geschlossen ist und an einen dem geschlossenen Schlauchende gegenüberliegenden (hinteren) Schlauchende eine Gewebeschlauchöffnung 22 aufweist. Der Grundkörper 20 verfügt über einen Sensorabschnitt 25 mit Sensoren 15 (die nur symbolisch angedeutet sind) und einen Halteabschnitt 30 mit einer ersten Strickstruktur 32, welche ein sitzfestes Halten des Grundkörpers 20 am Prothesenteil ermöglicht. Der Sensorabschnitt 25 weist eine der ersten Strickstruktur 32 unterschiedliche Strickstruktur 27 auf, welche ein sicheres Positionieren der Sensoren 15 relativ zum Prothesenteil ermöglicht. Im Sensorabschnitt 25 ist eine Sensorschicht 28 angeordnet, in der die Sensoren 15 strukturiert bzw. systematisch angeordnet sind. Die Strickstruktur 27 besteht aus einer widerstandsfähigen Kunstfaser, deren Fadenstärke so ausgelegt ist, die Sensoren 15 langfristig zu schützen. Dabei weist die erste Strickstruktur 32 des Halteabschnitts eine Fadenstärke auf, welche unterschiedlich zur Fadenstärke der Strickstruktur 27 des Sensorabschnitts ist.

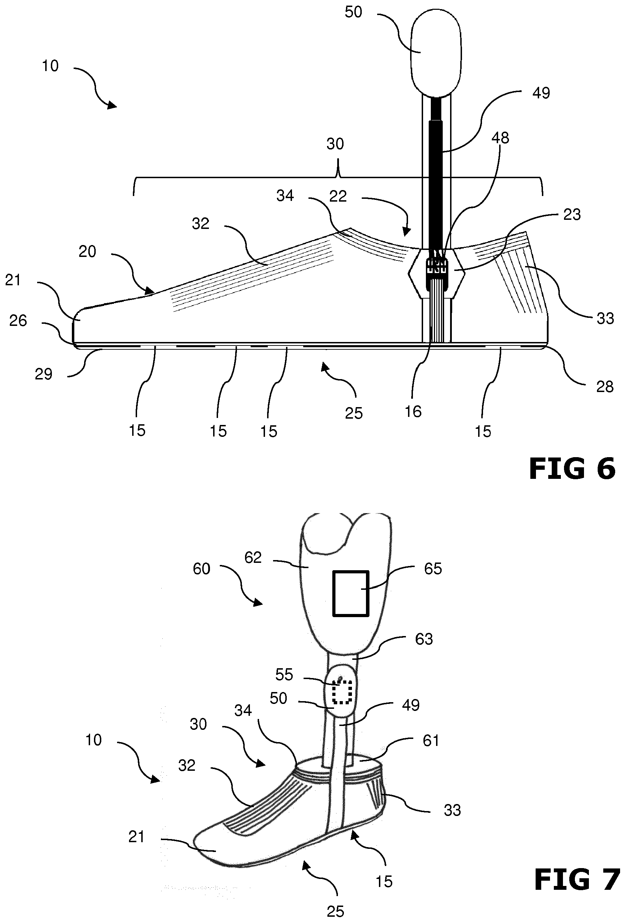

Fig. 2 zeigt die in dem Sensorabschnitt 25 des Sensorträgers 10 angeordneten Sensorschicht 28. Die Sensorschicht 28 weist in diesem Ausführungsbeispiel die Form einer Fusssohle auf. Dabei sind die Sensoren 15 an unterschiedlichen Positionen der Sensorschicht 28 angeordnet. Dargestellt sind vier Sensoren 15, wobei ein Sensor 15a im Bereich der Ferse, zwei Sensoren 15b und 15c im Bereich des Sohlenballens und ein Sensor 15d im Bereich der Zehen an der Fusssohle angeordnet sind. Die Sensoren 15 werden mithilfe der Strickstruktur 27 in dem Sensorabschnitt gehalten. Die Fadenstärke der Strickstruktur 27 liegt bei etwa 0.8 mm Die Sensoren 15 sind Drucksensoren und/oder Kraftsensoren, welche bei der Ausübung einer Druckkraft ein der Druckkraft proportionales elektrisches Signal erzeugen. Die Sensoren 15 sind mit einer elektrischen Leitung verbunden (siehe Fig. 6), mit welcher das elektrische Signal aus der Sensorschicht 28 transportiert werden kann.

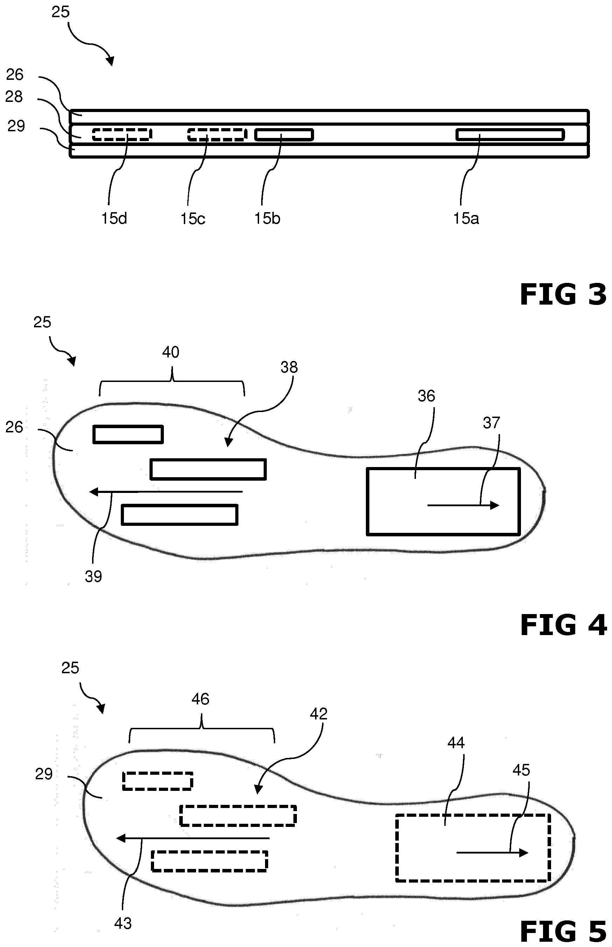

Fig. 3 zeigt den Sensorabschnitt 25, welcher mehrlagig ausgebildet ist. Die Sensorschicht 28 mit den darin eingebetteten Sensoren 15 weist an einer Seite eine innere Schutzschicht 26 und an der gegenüberliegenden Seite eine äussere Schutzschicht 29 auf. Die beiden Schutzschichten 26 bzw. 29 schützen die dazwischenliegende Sensorschicht 28, sodass eine Faltenbildung in der Sensorschicht 28 verhindert werden kann. Die Sensorschicht 28 ist - wenigstens peripher - an der äusseren Schutzschicht 29 festgeklebt.

Fig. 4 zeigt die innere Schutzschicht 26 des Sensorabschnitts 25. Diese innere Schutzschicht 26 ist im Anwendungsfall einer Beinprothese zugewandt und berührt dabei den Prothesenteil. Die innere Schutzschicht 26 ist abschnittsweise aus einem rutschhemmenden Material 36 gefertigt, welches ein Verrutschen der inneren Schicht 26 in eine erste Richtung 37 verhindert. Bei einer Fusssohle ist dieses rutschhemmende Material 36 beispielsweise im Bereich der Ferse angeordnet. Die innere Schutzschicht 26 ist abschnittsweise aus einem rutschfördernden Material 38 gefertigt, welches ein Verrutschen der inneren Schutzschicht 26 in eine zweite Richtung 39 verbessert. Dabei kann die erste Richtung 37 idealerweise gegenläufig zur zweiten Richtung 39 verlaufen. Bei einer Fusssohle ist dieses rutschfördernde Material 38, beispielsweise im Bereich des Fussballens angeordnet. Das rutschfördernde Material 38 ist als mehrteilige Profilstruktur 40 ausgebildet, wobei deren Teile einen strukturierten, funktionellen Abstand zueinander aufweisen. Beispielsweise bestehen das rutschhemmende Material und das rutschfördernde Material aus einem und demselben Material, welches die jeweilige Eigenschaft in unterschiedlichen Richtungen aufweist.

Fig. 5 zeigt die äussere Schutzschicht 29 des Sensorabschnitts 25. Diese äussere Schutzschicht 29 ist im Anwendungsfall der Prothese abgewandt und berührt dabei den Untergrund, an dem sich die Prothese im Anwendungsfall abstützt. Die äussere Schutzschicht 29 ist abschnittsweise aus einem rutschhemmenden Material 42 gefertigt, welche ein Verrutschen der äussere Schutzschicht 29 am Untergrund in eine erste Richtung 43 verhindert. Bei einer Fusssohle ist dieses rutschhemmende Material 42 beispielsweise im Bereich des Fussballens angeordnet. Die äussere Schutzschicht 29 ist abschnittsweise aus einem rutschfördernden Material 44 gefertigt, welche ein Verrutschen der äussere Schutzschicht 29 an dem Untergrund in eine zweite Richtung 45 verbessert. Dabei kann die erste Richtung 43 idealerweise gegenläufig zur zweiten Richtung 45 verlaufen. Bei einer Fusssohle ist dieses rutschfördernde Material 44, beispielsweise im Bereich der Ferse angeordnet.

Das rutschhemmende Material 42 ist hier als mehrteilige Profilstruktur 46 ausgebildet, wobei deren Teile einen funktionellen, strukturierten Abstand zueinander aufweisen.

Beispielsweise bestehen das rutschhemmende Material und das rutschfördernde Material aus ein und demselben Material, welches die jeweilige Eigenschaft in unterschiedlichen Richtungen aufweist.

Fig. 6 zeigt den Sensorträger 10, welcher hier zusätzlich mit einer Steuerungsvorrichtung 50 elektrisch verbunden ist. Der Grundkörper 20 umfasst den Gewebeschlauch 21 mit einer Gewebeschlauchöffnung 22. Der Grundkörper 20 weist einen Halteabschnitt 30 auf, der neben seiner ersten Strickstruktur 32 eine zweite Strickstruktur 33 aufweist, welche unterschiedlich ausgebildet sind. Die Fadenstärke der ersten Strickstruktur 32 liegt bei etwa 0.3 mm. Die zweite Strickstruktur 33 ist formstabil und umfasst den Fersenteil einer im Anwendungsfall im Gewebeschlauch 21 angeordneten Kunstfusses. Die Fadenstärke der zweiten Strickstruktur 33 liegt bei etwa 1 mm. Weiters weist der Halteabschnitt eine dritte Strickstruktur 34 auf, welche elastisch ausgebildet ist und eine Fadenstärke von etwa 0.6 mm aufweist. Die Strickstrukturen 33, 33, 34 werden mit Kunstfaserfäden gestrickt. Der Sensorabschnitt 25 umfasst die Sensoren 15, welche mit der Steuerungsvorrichtung 50 elektrisch verbunden sind. Dafür ist jeder Sensor 15 mit einer elektrischen Leitung 16 verbunden, welche jeden Sensor 15 mit der Steuerungsvorrichtung 50 zum Austauschen von elektrischen Signalen verbinden. Die elektrischen Signale werden bei der Detektion einer externen Druckkraft am jeweiligen Sensor 15 erzeugt und von diesem Sensor 15 an die Steuerungsvorrichtung 50 weitergeleitet und dort weiterverarbeitet. Die elektrischen Leitungen 16 verlaufen zunächst in der Sensorschicht 26 und verlassen die Sensorschicht 26 und verlaufen entlang des Gewebeschlauchs 21 in Richtung zur Gewebeschlauchöffnung 22. Der Gewebeschlauch 21 weist im Bereich der Gewebeschlauchöffnung 22 einen Verbindungsabschnitt 23 (Interface) auf. Am Verbindungsabschnitt 23 ist eine Schnittstelle 48 angeordnet, an der die elektrischen Leitungen 16 separierbar mit der Steuerungsvorrichtung 50 verbunden sind. Die Steuerungsvorrichtung 50 ist an einem Leitungsband 49 (Flachleiter) angeordnet, welches elektrische Leitungen aufweist. Die elektrischen Leitungen des Leitungsband 49 verbinden die Steuerungsvorrichtung 50 mit der Schnittstelle 48, sodass die elektrischen Signale der Sensoren 15 mit der Steuerungsvorrichtung 50 austauschbar sind. Das Leitungsband 49 ist von der Schnittstelle 48 separierbar, womit die Steuerungsvorrichtung 50 separierbar mit den Sensoren 15 verbunden ist.

Fig. 7 zeigt den Sensorträger 10 an einem Kunstfuss 61 einer Prothese 60. Der Kunstfuss 61 ist mithilfe eines Verbindungselements 62 - wie an sich bekannt - mit einem Prothesenschaft 62 mechanisch verbunden. Beim Anordnen des Kunstfusses 61 im Sensorträger 10 wird dieser in den Gewebeschlauch 21 eingeführt und von den Strickstrukturen 32, 33, 34 des Halteabschnitts 30 kraftschlüssig gehalten. Die Sohle des Kunstfusses 61 liegt an dem Sohlenabschnitt 25 des Sensorträgers 10 an. Somit liegt die Sohle des Kunstfusses 61 im Betriebszustand indirekt an den Sensoren 15 des Sensorträgers. Die Steuerungsvorrichtung 50 ist mithilfe einer Magnethalterung 55 lösbar am Verbindungselement 63 der Prothese 60 angeordnet.

Die Magnethalterung 55 bewirkt, dass sich die Steuerungsvorrichtung 50 relativ leicht und zerstörungsfrei vom Verbindungselement 63 lösen kann - z.B., wenn ein Benutzer unabsichtlich wo anstösst.

Die Steuerungsvorrichtung 50 ist elektrisch mit Stimulatoren 65 verbunden, welche an einem Prothesenschaft 62 der Prothese 60 angeordnet oder anordenbar sind. Damit wird gewährleistet, dass die elektrischen Signale der Sensoren 15 in der Steuerungsvorrichtung 50 verarbeitet werden und zum Erregen der jeweiligen Stimulatoren 65 verwendet werden können.

### Bezugszeichenliste

- 10: Sensorträger
- 15: Sensoren
- 16: elektrische Leitungen für 15
- 20: Grundkörper
- 21: Gewebeschlauch
- 22: Gewebeschlauchöffnung
- 23: Verbindungsabschnitt
- 25: Sensorabschnitt
- 26: innere Schutzschicht
- 27: Strickstruktur von 25
- 28: Sensorschicht
- 29: äussere Schutzschicht
- 30: Halteabschnitt
- 32: erste Strickstruktur von 30
- 33: zweite Strickstruktur von 30
- 34: dritte Strickstruktur von 30
- 36: rutschhemmendes Material
- 37: erste Richtung
- 38: rutschförderndes Material
- 39: zweite Richtung
- 40: Profilstruktur von 26
- 42: rutschhemmendes Material
- 43: erste Richtung
- 44: rutschförderndes Material
- 45: zweite Richtung
- 46: Profilstruktur von 29

- 48: Schnittstelle
- 49: Leitungsband
- 50: Steuerungsvorrichtung
- 55: Magnethalterung
- 60: Prothese
- 61: Kunstfuss
- 62: Prothesenschaft
- 63: Verbindungselement
- 65: Stimulatoren

## Patentansprüche

1. Sensorträger (10) zum Anordnen an einer Prothese, insbesondere an einer Kunsthand oder einem Kunstfuss, umfassend einen Grundkörper (20) zum Aufnehmen von Sensoren (15), wobei der Grundkörper (20) einen Sensorabschnitt (25) mit Sensoren (15) aufweist, und einen Halteabschnitt (30) zum Halten des Grundkörpers an (20) einem Prothesenteil aufweist, **dadurch gekennzeichnet, dass** der Grundkörper (20) einen gestrickten Gewebeschlauch (21) aufweist, und wobei der Halteabschnitt (30) eine erste Strickstruktur (32) aufweist, welche ein sitzfestes Halten des Grundkörpers (20) am Prothesenteil ermöglicht, und dass der Sensorabschnitt (25) eine davon unterschiedliche Strickstruktur (27) aufweist, welche ein sicheres Positionieren der Sensoren (15) relativ zum Prothesenteil ermöglicht.

2. Sensorträger nach Anspruch 1, **dadurch gekennzeichnet, dass** der Halteabschnitt (30) neben seiner ersten Strickstruktur (32) eine zweite Strickstruktur (33) aufweist, welche unterschiedlich zur ersten Strickstruktur (32) ausgebildet ist und insbesondere formstabil zum Umfassen eines Prothesenteil ausgebildet ist.

3. Sensorträger nach Anspruch 2, **dadurch gekennzeichnet, dass** der Halteabschnitt (30) neben seiner ersten Strickstruktur (32) eine dritte Strickstruktur (34) aufweist, welche bevorzugt unterschiedlich zur ersten Strickstruktur (32) und zweiten Strickstruktur (33) ausgebildet ist und insbesondere elastisch ausgebildet ist.

4. Sensorträger nach Anspruch 2 oder 3, **dadurch gekennzeichnet, dass** die erste Strickstruktur (32) des Halteabschnitts (30) eine erste Fadenstärke aufweist und die zweite Strickstruktur (33) des Halteabschnitts (30) eine zweite Fadenstärke aufweist und bevorzugt die dritte Strickstruktur (34) des Halteabschnittes (30) eine dritte Fadenstärke aufweist.

5. Sensorträger nach einem der Ansprüche 1 bis 4, **dadurch gekennzeichnet, dass** die Strickstruktur (27) des Sensorabschnitts (25) eine Fadenstärke aufweist, welche unterschiedlich zur ersten Fadenstärke des Halteabschnitts (30) ist.

6. Sensorträger nach einem der Ansprüche 1 bis 5, **dadurch gekennzeichnet, dass** zumindest eine der Strickstrukturen (27, 32, 33, 34) aus einer widerstandsfähigen Kunstfaser hergestellt ist.

7. Sensorträger nach einem der Ansprüche 1 bis 6, **dadurch gekennzeichnet, dass** der Sensorabschnitt (25) mehrlagig ausgebildet ist und eine Sensorschicht (28) aufweist, in der die Sensoren (15) eingebettet sind, wobei die Sensoren bevorzugt zumindest einen Drucksensor und/oder zumindest einen Kraftsensor umfassen.

8. Sensorträger nach einem der Ansprüche 1 bis 7, **dadurch gekennzeichnet, dass** an dem Sensorabschnitt (25) zusätzlich zur Strickstruktur (27) eine im Anwendungszustand an einem Prothesenteil abgewandte, äussere Schutzschicht (29) vorgesehen ist und bevorzugt eine dem Prothesenteil zugewandte innere Schutzschicht (26) vorgesehen ist.

9. Sensorträger nach Anspruch 8, **dadurch gekennzeichnet, dass** die äussere Schutzschicht (29) und/oder die innere Schutzschicht (26) zumindest in eine Richtung rutschhemmend ausgebildet ist/sind, insbesondere zumindest abschnittsweise aus rutschhemmenden Material gefertigt ist/sind und/oder zumindest in eine Richtung rutschfördernd ausgebildet ist/sind, insbesondere zumindest abschnittsweise aus rutschförderndem Material gefertigt ist/sind und bevorzugt eine Profilstruktur zum Verbessern der rutschhemmenden oder rutschfördernden Eigenschaft aufweist.

10. Sensorträger nach einem der Ansprüche 8 bis 9, **dadurch gekennzeichnet, dass** die Sensorschicht (28) zwischen der äusseren Schutzschicht (29) und der inneren Schutzschicht (26) eingebettet ist.

11. Sensorträger nach einem der Ansprüche 7 bis 10, **dadurch gekennzeichnet, dass** die Sensorschicht (28) stoffschlüssig im Sensorabschnitt (25) angeordnet ist.

12. Sensorträger nach einem der Ansprüche 1 bis 11, **dadurch gekennzeichnet, dass** die Sensoren (15) des Sensorträgers mit einer Steuerungsvorrichtung (50) elektrisch verbunden sind oder verbindbar sind.

13. Sensorträger nach Anspruch 12, **dadurch gekennzeichnet, dass** die Steuerungsvorrichtung (50) separierbar mit den Sensoren (15) der Sensorschicht (28) verbunden ist.

14. Sensorträger nach Anspruch 12 oder 13, **dadurch gekennzeichnet, dass** die Steuerungsvorrichtung (50) am Prothesenteil bevorzugt lösbar befestigbar ist.

15. Sensorträger nach Anspruch 14, **dadurch gekennzeichnet, dass** die Steuerungsvorrichtung (50) mittels einer Magnethalterung (55) am Prothesenteil befestigbar ist.

## Claims

1. A sensor support (10) for arranging on a prosthesis, particularly on an artificial hand or an artificial foot, comprising a main part (20) for receiving sensors (15), wherein said main part (20) has a sensor section (25) with sensors (15), and a holding section (30) for holding the main part (20) on a prosthesis part, **characterized in that** the main part (20) has a knitted fabric tube (21), and wherein the holding section (30) has a first knitted structure (32) which allows a secure hold of the main part (20) on the prosthesis part, and that the sensor section (25) has a different knitted structure (27) which allows secure positioning of the sensors (15) relative to the prosthesis part.

2. The sensor support according to Claim 1, **characterized in that** besides its first knitted structure (32) the holding section (30) has a second knitted structure (33) which is of a different structure from the first knitted structure (32) and in particular is constructed in dimensionally stable manner to comprise a prosthesis part.

3. The sensor support according to Claim 2, **characterized in that** besides its first knitted structure (32) the holding section (30) has a third knitted structure (34) which is preferably of a different structure from the first knitted structure (32) and the second knitted structure (33) and in particular is constructed elastically.

4. The sensor support according to Claim 2 or 3, **characterized in that** the first knitted structure (32) of the holding section (30) has a first denier, and the second knitted structure (33) of the holding section (30) has a second denier, and the third knitted structure (34) of the holding section (30) preferably has a third denier.

5. The sensor support according to any one of Claims 1 to 4, **characterized in that** the knitted structure (27) of the sensor section (25) has a denier which is different from the first denier of the holding section (30).

6. The sensor support according to any one of Claims 1 to 5, **characterized in that** at least one of the knitted structures (27, 32, 33, 34) is produced from a robust synthetic fibre.

7. The sensor support according to any one of Claims 1 to 6, **characterized in that** the sensor section (25) is of multilayer construction and includes a sensor layer (28) in which the sensors (15) are embedded, wherein the sensors preferably comprise at least one pressure sensor and/or at least one force sensor.

8. The sensor support according to any one of Claims 1 to 7, **characterized in that** on the sensor section (25), in addition to the knitted structure (27) an outer protection layer (29) is provided facing away from a prosthesis part in the application state, and preferably an inner protection layer (26) is provided facing towards the prosthesis part.

9. The sensor support according to Claim 8, **characterized in that** the outer protection layer (29) and/or the inner protection layer (26) is/are constructed to suppress slipping in at least one direction, in particular said protection layer(s) is/are constructed at least with sections made from a non-slip material, and/or is/are constructed to enhance slipping at least in one direction, in particular said protection layer(s) is/are constructed at least with sections made from a slip enhancing material and preferably has a profile structure for improving the non-slip or slip enhancing property.

10. The sensor support according to one of Claims 8 to 9, **characterized in that** the sensor layer (28) is embedded between the outer protection layer (29) and the inner protection layer (26).

11. The sensor support according to any one of Claims 7 to 10, **characterized in that** the sensor layer (28) is arranged in the sensor section (25) and materially bonded therewith.

12. The sensor support according to any one of Claims 1 to 11, **characterized in that** the sensors (15) of the sensor support are or can be electrically connected to a control apparatus (50).

13. The sensor support according to Claim 12, **characterized in that** the control apparatus (50) is connected separably to the sensors (15) of the sensor layer (28).

14. The sensor support according to Claim 12 or 13, **characterized in that** the control apparatus (50) can be fastened preferably separately to the prosthesis part.

15. The sensor support according to Claim 14, **characterized in that** the control apparatus (50) can be fastened to the prosthesis part by means of a magnetic fixture (55).

## Revendications

1. Support de capteurs (10) destiné à être agencé sur une prothèse, en particulier sur une main artificielle ou un pied artificiel, comprenant un corps de base (20) pour recevoir des capteurs (15), dans lequel le corps de base (20) présente un tronçon de capteurs (25) avec des capteurs (15), et un tronçon de maintien (30) pour maintenir le corps de base (20) sur une pièce de prothèse, **caractérisé en ce que** le corps de base (20) présente un flexible tissé (21) tricoté, et dans lequel le tronçon de maintien (30) présente une première structure tricotée (32), qui permet un maintien fixe du corps de base (20) sur la pièce de prothèse, et que le tronçon de capteurs (25) présente une structure tricotée (27) différente de celle-ci qui permet un positionnement sûr des capteurs (15) par rapport à la pièce de prothèse.

2. Support de capteurs selon la revendication 1, **caractérisé en ce que** le tronçon de maintien (30) présente une deuxième structure tricotée (33) en plus de sa première structure tricotée (32), laquelle est formée différemment de la première structure tricotée (32) et est conçue en particulier avec une forme stable pour contenir une pièce de prothèse

3. Support de capteurs selon la revendication 2, **caractérisé en ce que** le tronçon de maintien (30) présente une troisième structure tricotée (34) en plus de sa première structure tricotée (32), laquelle est conçue de préférence différemment de la première structure tricotée (32) et de la deuxième structure tricotée (33) et est notamment conçue élastique.

4. Support de capteurs selon la revendication 2 ou 3, **caractérisé en ce que** la première structure tricotée (32) du tronçon de maintien (30) présente une première épaisseur de fil et la deuxième structure tricotée (33) du tronçon de maintien (30) présente une deuxième épaisseur de fil, et de préférence la troisième structure tricotée (34) du tronçon de maintien (30) présente une troisième épaisseur de fil.

5. Support de capteurs selon l'une des revendications 1 à 4, **caractérisé en ce que** la structure tricotée (27) du tronçon de capteurs (25) présente une épaisseur de fil qui est différente de la première épaisseur de fil du tronçon de maintien (30).

6. Support de capteurs selon l'une des revendications 1 à 5, **caractérisé en ce qu'**au moins une des structures tricotées (27, 32, 33, 34) est fabriquée dans une fibre artificielle résistante.

7. Support de capteurs selon l'une des revendications 1 à 6, **caractérisé en ce que** le tronçon de capteurs (25) est conçu en plusieurs couches et présente une couche de capteurs (28) dans laquelle les capteurs (15) sont enchâssés, dans lequel les capteurs comprennent de préférence au moins un capteur de pression et/ou au moins un capteur de force.

8. Support de capteurs selon l'une des revendications 1 à 7, **caractérisé en ce que** sont prévues en plus de la structure tricotée (27) sur le tronçon de capteurs (25), une couche de protection (29) extérieure détournée d'une pièce de prothèse dans l'état d'utilisation, et de préférence, une couche de protection intérieure (26) tournée vers la pièce de prothèse.

9. Support de capteurs selon la revendication 8, **caractérisé en ce que** la couche de protection extérieure (29) et/ou la couche de protection intérieure (26) est/sont conçue(s) anti-glissante(s) au moins dans un sens, est/sont fabriquée(s) en matériau anti-glissement au moins par tronçons, est/sont conçue(s) en favorisant le glissement au moins dans un sens, est/sont fabriquée(s) en matériau favorisant le glissement au moins par tronçons et présente(nt) de préférence une structure profilée pour améliorer la propriété anti-glissement ou favorisant le glissement.

10. Support de capteurs selon l'une des revendications 8 à 9, **caractérisé en ce que** la couche de capteurs (28) est enchâssée entre la couche de protection extérieure (29) et la couche de protection intérieure (26).

11. Support de capteurs selon l'une des revendications 7 à 10, **caractérisé en ce que** la couche de capteurs (28) est disposée en complément de matière dans le tronçon de capteurs (25).

12. Support de capteurs selon l'une des revendications 1 à 11, **caractérisé en ce que** les capteurs (15) du support de capteur sont reliés ou peuvent être reliés électriquement à un dispositif de commande (50) .

13. Support de capteurs selon la revendication 12, **caractérisé en ce que** le dispositif de commande (50) est relié de manière séparable aux capteurs (15) de la couche de capteurs (28).

14. Support de capteurs selon la revendication 12 ou 13, **caractérisé en ce que** le dispositif de commande (50) peut être fixé sur la pièce de prothèse en étant de préférence détachable.

15. Support de capteurs selon la revendication 14, **caractérisé en ce que** le dispositif de commande (50) peut être fixé sur la pièce de prothèse au moyen d'une fixation magnétique (55).
